# EUROPEAN PATENT SPECIFICATION

(11) **EP 0 422 938 B1**
(45) Date of publication and mention of the grant of the patent: **22.02.1995**
(21) Application number: 90311151.6
(22) Date of filing: 11.10.1990
(51) Int. Cl.: C07K 5/10, C07K 14/75

(54) **Fibrinogen receptor antagonists**
Fibrinogen-Rezeptor-Antagonisten
Antagonistes du récepteur du fibrinogène

(30) Priority: 13.10.1989 US 421224
(43) Date of publication of application: 17.04.1991
(73) Proprietor: Merck & Co., Inc., Rahway New Jersey 07065-0900 (US)
(72) Inventor: Nutt, Ruth F., Green Lane, PA 18054 (US); Brady, Stephen F., Philadelphia, PA 19118 (US); Veber, Daniel F., Ambler, PA 19002 (US); Duggan, Mark E., Wynnewood, PA 19096 (US)
(74) Representative: Cole, William Gwyn

(56) References cited:
- EP-A- 0 275 748

## Description

### BACKGROUND OF THE INVENTION

This invention relates to compounds for inhibiting the binding of fibrinogen to blood platelets, and for inhibiting the aggregation of blood platelets.

Fibrinogen is a glycoprotein, present in blood plasma, which participates in platelet aggregation and fibrin formation. Platelets are cell-like anucleated fragments, found in the blood of all mammals, which participate in blood coagulation. Interaction of fibrinogen with a receptor on the platelet membrane glycoprotein complex IIb/IIIa is known to be essential for normal platelet function. Zimmerman et al., U.S. Patent No. 4,683,291, describes peptides having utility in the study of fibrinogen-platelet, platelet-platelet, and cell-cell interactions. The peptides are described as having utility where it is desirable to retard or prevent formation of a thrombus or clot in the blood. The general formula for the peptides is:
H₂N-(Ch)-Arg-Gly-Asp-(Cx)-H
where Ch and Cx are sequences of amino acids.

Pierschbacher et al., U.S. Patent No. 4,589,881, describes the sequence of an 11.5 kDal polypeptide fragment of fibronectin which embodies the cell-attachment-promoting activity of fibronectin. A specifically described fragment is:
H-Tyr-Ala-Val-Thr-Gly-Arg-Gly-Asp-
Ser-Pro-Ala-Ser-Ser-Lys-Pro-Ile-
Ser-Ile-Asn-Tyr-Arg-Thr-Glu-Ile-
Asp-Lys-Pro-Ser-Gln-Met-OH
Ruoslahti et al., U.S. Patent No. 4,614.517, describes tetrapeptides which alter cell-attachment activity of cells to various substrates. The peptides are stated to "consist essentially of" the following sequence:
X-Arg-Gly-Asp-Ser-Y
wherein X is H or one or amino acids and Y is OH or one or amino acids. Figure 1 lists the polypeptides that were synthesized by Ruoslahti et al. in "determining the smallest peptide exhibiting cell attachment activity".
Ruoslahti et al., U.S. Patent No. 4,578,079, describes similar tetrapeptides having Ser substituted with Thr or Cys.
Pierschbacher et al., Proc. Natl. Acad. Sci. USA, Vol. 81, pp.5985-5988, October 1984 describe variants of the cell recognition site of fibronectin that retain attachment-promoting activity. They assayed the cell attachment-promoting activities of a number of structures closely resembling the Arg-Gly-Asp-Ser peptide, and found "that the arginine, glycine, and aspartate residues cannot be replaced even with closely related amino acids, but that several amino acids can replace serine without loss of activity."
Ruoslahti et al., Science, Vol. 238, pp. 491-497, October 23, 1987, discuss cell adhesion proteins. They specifically state that "[e]lucidation of the amino acid sequence of the cell-attachment domain in fibronectin and its duplication with synthetic peptides establish the sequence Arg-Gly-Asp (RGD) as the essential structure recognized by cells in fibronectin".
Cheresh, Proc. Natl. Acad. Sci. USA, Vol. 84, pp. 6471-6475, September 1987, describes the Arg-Gly-Asp-directed adhesion receptor involved in attachment to fibrinogen and von Willebrand Factor. Adams et al., U. S. Patent No. 4,857,508, describes tetrapeptides which inhibit platelet aggregation and the formation of a thrombus. The tetrapeptides have the formula:
X-Gly-Asp-Y
wherein X can be H2NC(=NH)NH(CH2)nCH(Z)COOH or Ac-Arg, wherein Z = H, NH2, or NH-Acyl and n=1-4, and wherein Y can be Tyr-NH2, Phe-NH2 or a group of a specifically defined formula.

Applicants have discovered fibrinogen receptor antagonists which do not contain the amino acid sequence Arg-Gly-Asp which is taught in the art as specifically required for binding to platelet membrane glycoprotein complex IIb/IIIa.

### SUMMARY OF THE INVENTION

Compounds of the present invention inhibit binding of fibrinogen to the platelet membrane glycoprotein complex IIb/IIIa receptor and contain an amino acid sequence:
XX-Gly-Asp
wherein XX is a synthetic alpha amino acid containing a linear side-chain. 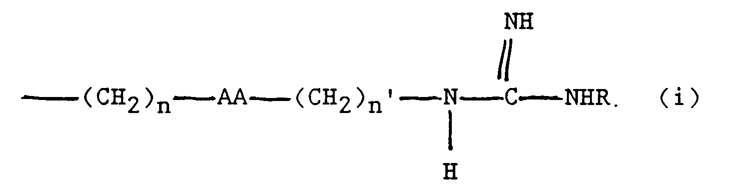
wherein n + n' is 3; AA is a single bond; and R is phenyl or benzyl;
or

―(CH₂)ₙ―AA―(CH₂)ₙ'―NHR (ii)

wherein:
n is 1,2,3 or 4;
n' is 2,3 or 4;
AA is an oxygen atom, a sulfur atom, or a single bond; and
R is H, C₁₋₆ alkyl, substituted or unsubstituted aryl, substituted or unsubstituted arylmethyl or substituted or unsubstituted cycloalkyl, provided that when AA is a single bond and R is H, then n+n' is not 4 or 3.
These compounds are surprising in view of the prior art which teaches that the sequence Arg-Gly-Asp is required in order to achieve binding to the IIb/IIIa receptor.
Preferred compounds of the invention are those having selectivity over other integrin receptors. The preferred compounds include those wherein XX is a synthetic alpha amino acid containing an amino group linear side chain, as represented above by (ii).

The present invention is a fibrinogen receptor antagonist having the following structure: 
Z Z
XX-Gly-Asp
wherein XX represents a synthetic α-amino acid as defined below and ZZ represents a sequence of 1, 2, 3, or 4 amino acids as defined below.
XX shares an amide bond with Gly and an amide bond with ZZ, and is defined as having a side chain X 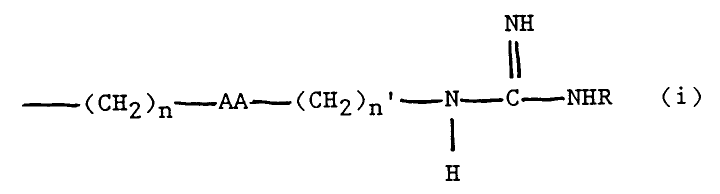
wherein n + n' is 3; AA is a single bond; and R is phenyl or benzyl;
or

―(CH₂)ₙ―AA―(CH₂)ₙ'―NHR (ii)

wherein:
n is 1,2,3 or 4;
n' is 2,3 or 4;
AA is an oxygen atom, a sulfur atom, or a single bond; and
R is H, C₁₋₆ alkyl, substituted or unsubstituted aryl, substituted or unsubstituted arylmethyl or substituted or unsubstituted cycloalkyl, provided that when AA is a single bond and R is H, then n+n' is not 4 or 3.
Preferably, when X is defined by (ii), then n+n' is 5, AA is a single bond and R is H.
ZZ is defined as follows: 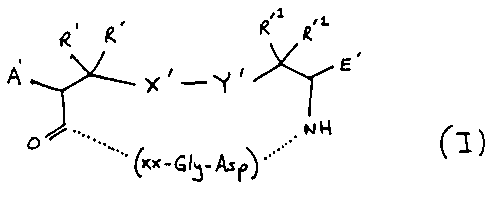
wherein:
A′ is H, acylamido, acylaminoacylamido, acylamino-N-methylamino-acylamido;
R′ and R'¹ are independently H, methyl, ethyl or a lower alkyl group having 1 to 5 carbons;
X′-Y′ is S-S, CH₂-S, S-CH₂, CH₂CH₂, CH₂, CH₂CH₂CH₂, CH₂-S-S, CH₂-S-S-CH₂, S-S-CH₂; and
E′ is H, COOH, CONH₂, CONHR², CONR³R⁴, CH₂OH,CO₂R^{2,}CH₃ wherein R² is an alkyl group having 1 to 4 carbon atoms, R³R⁴ is an alkyl group having 1 to 4 carbon atoms or NR³R⁴ is a secondary amino acid, or 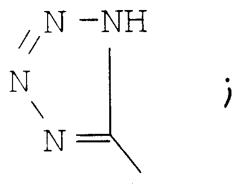
or ZZ is 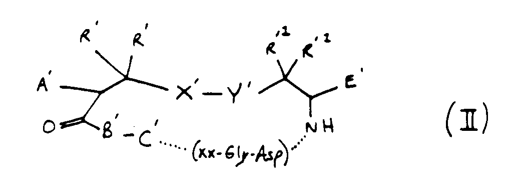
wherein:
A′ is as defined above;
R′ and R'¹ are as defined above;
X′ - Y′ is as defined above;
B′ is a D- or L- α-amino acid;
C′ is a D- or L- secondary α-amino acid preferably selected from proline, β- methylproline, β,β- dimethylproline, gamma-hydroxyproline, anhydroproline, thioproline, β - methylthioproline, β,β - dimethylthioproline, pipecolic acid, azetidine carboxylic acid and an N-methyl amino acid, or a D-or L- primary α-amino acid; and
E′ is as defined above;
or ZZ is 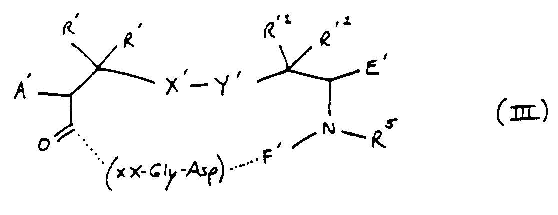
wherein:
A′ is as defined above;
R′ and R'¹ are as defined above;
X′ - Y′ are as defined above;
E′ is as defined above;
F′ is an L-amino acid, preferably an L-amino acid selected from tryptophan, phenylalanine, leucine, valine, isoleucine, α-naphthylalanine, β-naphthylalanine, methionine, tyrosine, arginine, lysine, homoarginine, ornithine, histidine, substituted tryptophan, substituted phenylalanine or substituted tyrosine; and R⁵ is H or methyl;
or ZZ is 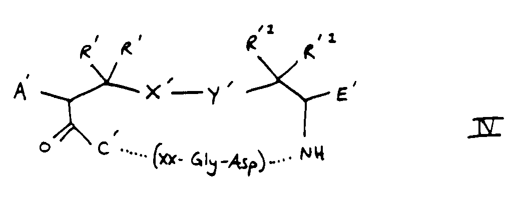
wherein
A′ is as defined above;
R′ and R'¹ are as defined above;
X′-Y′ is as defined above;
C′ is as defined above; and
E′ is as defined above.
or ZZ is 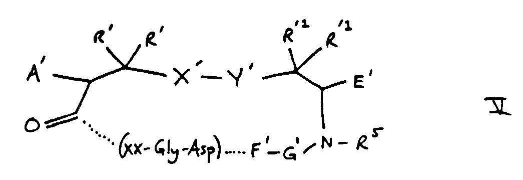
wherein
A' is as defined above;
R' and R'¹ are as defined above;
X'-Y' is as defined above;
F' is as defined above;
G' is a D- or L-α-amino acid, secondary
cyclic amino acid, or N-methyl amino acid;
E' is as defined above; and
R⁵ is as defined above.
Exemplary compounds of the invention are:
Ac-(Arg(Ph))-Gly-Asp-Phe;
Ac-(Arg(Bzl))-Gly-Asp-Phe;
Aha-Gly-Asp-Phe;
Aha-Gly-Asp-Trp;

Ac-Cys-Asn-(DiMeTzl)-(homoLys)-Gly-Asp-Cys-OH;

Ac-Cys-(DiMeTzl)-(homoLys)-Gly-Asp-Cys-OH;

(GuaValA)-Gly-Asp-Phe;
(GuaValA)-Gly-Asp-Trp;
(GuaHexA)-Gly-Asp-Trp;
(GuaHepA)-Gly-Asp-Trp;
(7-AhepA)-Gly-Asp-Trp;
(8-AoctA)-Gly-Asp-Trp; 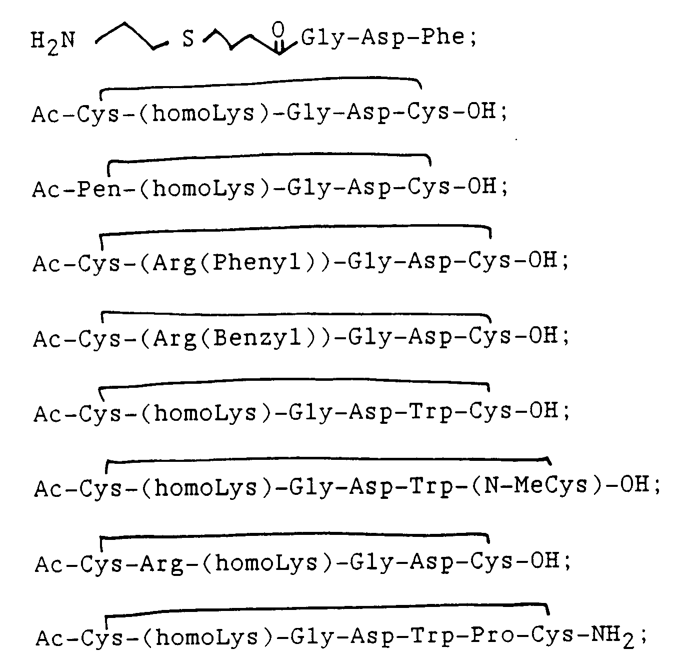
H2N S O Gly-Asp-Phe;

Ac-Cys-(homoLys)-Gly-Asp-Cys-OH;

Ac-Pen-(homoLys)-Gly-Asp-Cys-OH;

Ac-Cys-(Arg(Phenyl))-Gly-Asp-Cys-OH;

Ac-Cys-(Arg(Benzyl))-Gly-Asp-Cys-OH;

Ac-Cys-(homoLys)-Gly-Asp-Trp-Cys-OH;

Ac-Cys-(homoLys)-Gly-Asp-Trp-(N-MeCys)-OH;

Ac-Cys-Arg-(homoLys)-Gly-Asp-Cys-OH;

Ac-Cys-(homoLys)-Gly-Asp-Trp-Pro-Cys-NH2;
c((7-AhepA)-(homoLys)-Gly-Asp-Trp-Pro);
c((6-AhexA)-(homoLys)-Gly-Asp-Trp-Pro);
c((7-AhepA)-(homoLys)-Gly-Asp-(beta-Nal)-Pro);
c((7-AhepA)-(Arg(Phenyl))-Gly-Asp-Trp-Pro);
c((7-AhepA)-(Arg(Benzyl))-Gly-Asp-Trp-Pro); 
Ac-Cys-Asn-Pro-(homoLys)-Gly-Asp-Cys-OH;

Ac-Pen-Asn-(DiMeTzl)-(homoLys)-Gly-Asp-Cys-OH;

Ac-Cys-Asn-Pro-(Arg(Phenyl))-Gly-Asp-Cys-OH;

Ac-Cys-Asn-(DiMeTzl)-(Arg(Phenyl))-Gly-Asp-Cys-OH;

Ac-Cys-Asn-(DiMeTzl)-(Arg(Benzyl))-Gly-Asp-Cys-OH;
and
c(Aha-(homoLys)-Gly-Asp-Trp-Pro).
The preferred compounds are: Ac-Cys-Asn-(DiMetzl)-(homoLys)-Gly-Asp-Cys-OH;
and
c(Aha-(homoLys)-Gly-Asp-Trp-Pro).

In addition to the common three letter abbreviations used to identify common amino acids, applicants have used the following abbreviation designations:
- homoLys: homo-lysine
- Aha, 7-AhepA: 7-aminoheptanoic acid
- Arg(Ph): phenylarginine
- Arg(Bzl): benzylarginine
- DiMeTzl: dimethylthioproline
- AhexA: 6-aminohexanoic acid
- AoctA: 8-aminooctanoic acid
- GuaValA: 5-guanidovaleric acid
- GuaHexA: 6-guanidoxhexanoic acid
- GuaHepA: 7-guanidoheptanoic acid
- beta-Nal: beta-naphthylalanine

The invention also includes compositions, comprising fibrinogen receptor antagonist peptides of the present invention and one or more pharmacologically acceptable carriers, e.g. saline, at a pharmacologically acceptable pH, e.g. 7.4, which are suitable for continuous intravenous or oral or intravenous bolus administration for promoting inhibition of platelet aggregation.

The invention also includes methods for inhibiting platelet aggregation which comprise administering to a patient, either by continuous intravenous or oral or intravenous bolus method, an effective amount of a composition of the present invention.

### DETAILED DESCRIPTION OF THE INVENTION

Compounds of the invention are fibrinogen receptor antagonists which inhibit fibrinogen induced platelet aggregation. These compounds are prepared by solid phase synthesis which is well known in the art, or by liquid method well known in the art (Neurath, Hill & Boeder, Eds. "The Proteins" 3rd Edition, Vol. II, Academic Press, 1976).

The compounds of the invention are specifically useful for preventing formation of blood clots by inhibiting the binding of fibrinogen to the platelet membrane glycoprotein complex IIb/IIIa receptor. Preferred compounds have selectivity over other integrin receptors, and thus are specifically designed for preventing thrombosis.

The procedures for synthesizing synthetic amino acids defined by XX such as homolysine, 7-aminoheptanoic acid, phenylarginine, 5-guanidovaleric acid, and benzylarginine are well known in the art. Synthesis of DL-homoLys is described, for example, in Payne, Synthetic Comm. 15 (14), pp. 1277-1290 (1985). Guanylation procedures are described, for example, in Methods of Enzymology, 256,558 (1972), and in A.E. Miller and J.J. Bischoff, Synthesis, pp. 777-779 (1986).

Compounds of the invention may be prepared using solid phase peptide synthesis, such as that described by Merrifield, J. Am. Chem. Soc., 85, 2149 (1964), although other equivalent chemical syntheses known in the art can also be used, such as the syntheses of Houghten, Proc. Natl. Acad. Sci., 82, 5132 (1985). Solid-phase synthesis is commenced from the C-terminus of the peptide by coupling a protected amino acid to a suitable resin, as generally set forth in U.S. Patent No. 4,244,946, issued Jan. 21, 1982 to Rivier et al., the disclosure of which is hereby incorporated by reference. Solution method can be used as described by Neurath et al. Chapter 2, pp. 106-253. Examples of synthesis of this general type are set forth in U.S. Patent Nos. 4,305,872 and 4,316,891.

In synthesizing these polypeptides, the carboxyl terminal amino acid, having its alpha-amino group suitably protected, is covalently coupled to a chloromethylated polystyrene resin or the like, such as p-hydroxymethylphenylacetylamidomethylresin (PAM resin). The chloromethylated polystyrene resin is composed of fine beads (20-70 microns in diameter) of a synthetic resin prepared by copolymerization of styrene with 1 to 2 percent divinylbenzene. The benzene rings in the resin are chloromethylated in a Friedel-Crafts reaction with chloromethyl methyl ether and stannic chloride. The Friedel-Crafts reaction is continued until the resin contains 0.5 to 5 mmoles of chlorine per gram of resin. After removal of the alpha-amino protecting group, as by using trifluoroacetic acid in methylene chloride, the amino protected derivative of the next amino acid in the sequence is added along with a condensation coupling agent such as dicyclohexylcarbodiimide. The remaining alpha-amino and side-chain-protected amino acids are then coupled by condensation stepwise in the desired order to obtain an intermediate compound connected to the resin.

The condensation between two amino acids, or an amino acid and a peptide, or a peptide and a peptide can be carried out according to the usual condensation methods such as azide method, mixed acid anhydride method, DCC (dicyclohexyl-carbodiimide) method, BOP (benzotriazole-1-yloxytris (dimethylamino) phosphonium hexafluorophosphate method, active ester method (p-nitrophenyl ester method, N-hydroxysuccinimido ester method, cyanomethyl ester method, etc.), Woodward reagent K method, carbonyldiimidazol method, oxidation-reduction method. In the case of elongating the peptide chain in the solid phase method, the peptide is attached to an insoluble carrier at the C-terminal amino acid. For insoluble carriers, those which react with the carboxy group of the C-terminal amino acid to form a bond which is readily cleaved later, for example, halomethyl resin such as chloromethyl resin and bromomethyl resin, hydroxymethyl resin, aminomethyl resin, benzhydrylamine resin, and t-alkyloxycarbonylhydrazide resin can be used.

Common to chemical syntheses of peptides is the protection of the reactive side-chain groups of the various amino acid moieties with suitable protecting groups at that site until the group is ultimately removed after the chain has been completely assembled. Also common is the protection of the alpha-amino group on a amino acid or a fragment while that entity reacts at the carboxyl group followed by the selective removal of the alpha-amino-protecting group to allow subsequent reaction to take place at that location. Accordingly, it is common that, as a step in the synthesis, an intermediate compound is produced which includes each of the amino acid residues located in the desired sequence in the peptide chain with various of these residues having side-chain protecting groups. These protecting groups are then commonly removed substantially at the same time so as to produce the desired resultant product following purification.

The applicable protective groups for protecting the alpha-and omega-side chain amino groups are exemplified such as benzyloxycarbonyl (hereinafter abbreviated as Z), isonicotinyloxycarbonyl (iNOC), 0-chlorobenzyloxycarbonyl [Z(2-Cl)], p-nitrobenzyloxycarbonyl [Z(NO₂)],p-methoxybenzyloxycarbonyl [Z(OMe)],t-butoxycarbonyl (Boc), t-amyloxycarbonyl (Aoc), isobornyloxycarbonyl, adamantyloxycarbonyl, 2-(4-biphenyl)-2- propyloxycarbonyl (Bpoc),9-fluorenylmethoxycarbonyl (Fmoc), methylsulfonylethoxycarbonyl (Msc), trifluoroacetyl, phthalyl, formyl, 2-nitrophenylsulphenyl (NPS), diphenylphosphinothioyl (Ppt), dimethylphosphinothioyl (Mpt) and the like.

Protective groups for carboxy group include, for example, benzyl ester (OBzl), cyclohexyl ester (Chx) 4-nitrobenzyl ester (ONb), t-butyl ester (OBut), 4-pyridylmethyl ester (OPic), and the like. It is desirable that specific amino acids such as arginine, cysteine, and serine possesing a functional group other than amino and carboxyl groups are protected by a suitable protective group as occasion demands. For example, the guanidino group in arginine may be protected with nitro, p-toluenesulfonyl, benzyloxycarbonyl, adamantyloxycarbonyl, p-methoxybenzenesulfonyl, 4-methoxy-2, 6-dimethylbenzenesulfonyl (Mds), 1,3,5-trimethylphenylsulfonyl (Mts), and the like. The thiol group in cysteine may be protected with benzyl, p-methoxybenzyl, triphenylmethyl, acetylamidomethyl, ethylcarbamoyl, 4-methylbenzyl, 2,4,6-trimethylbenzyl (Tmb) etc., and the hydroxyl group in serine can be protected with benzyl, t-butyl, acetyl, tetrahydropyranyl etc.

Stewart and Young, "Solid Phase Peptide Synthesis:, Pierce Chemical Company, Rockford, IL (1984) provides detailed information regarding procedures for preparing peptides. Protection of α-amino groups is described on pages 14-18, and side-chain blockage is described on pages 18-28. A table of protecting groups for amine, hydroxyl and sulfhydryl functions is provided on pages 149-151. These descriptions are hereby incorporated by reference.

After the desired amino-acid sequence has been completed, the intermediate peptide is removed from the resin support by treatment with a reagent, such as liquid HF, which not only cleaves the peptide from the resin, but also cleaves all the remaining protecting groups from the side chain which do not interfere in the cyclization reaction. Potentially reactive side chains functionalities are protected with blocking groups which are stable to HF. The peptides are cyclized by any one of several known procedures (see Schroder and Lubke, "The Peptides: Methods of Peptide Synthesis" Vol. I, Academic Press, New York (1965), pp. 271-286, the contents of which are hereby incorporated by reference), e.g. by forming a disulfide bridge between the cysteine residues using iodine in AcOH, or air oxidation at pH 8 in dilute NH₄ OAc buffer. The polypeptide can then be purified by gel permeation chromatography followed by preparative HPLC, as described in Rivier et al., Peptides: Structure and Biological Function (1979) pp. 125-128.

### EXAMPLE 1

### Synthesis of Ac-Cys(Pmb)-Asn-(DiMeTzl)-(homoLys(Cbz))-Gly-Asp(Bzl) -Cys (Pmb)-O Pam Ⓡ and ultimately Ac-Cys-Asn-(DiMeTzl)-(homoLys)-Gly-Asp-Cys-OH

Starting with 
   PMB
    |
Boc-Cys-O-Pam
resin, the alpha-amino Boc protecting group (tert-butylcarbonyl) is removed (while the Cys side-chain remains protected by p-methylbenzyl) using trifluoroacetic acid and methylene chloride, and the α-deprotected cysteine neutralized with diisopropylethyl amine. Boc-protected Asp (benzyl) (Asp (Bzl)) is then coupled to cysteine mediated by dicyclohexyl-carbodiimide, and deprotected with trifluoroacetic acid and methylene chloride. Asp is then neutralized with diisopropylethylamine. Following this stepwise procedure of coupling with dicyclohexylcarbodiimide, deprotection with trifluoroacetic acid and methylene chloride, and neutralization with diisopropylethylamine, Boc-protected Gly, homoLys(Cbz) DiMeTzl, Asn, Cys(Pmb) residues are coupled in succession. The final Cys is then acetylated with acetic anhydride.

Following acetylation, the following peptide-resin is formed: 
PMB                          Cbz          Bzl PMB
Acetyl-Cys-Asn-(DiMeTzl)-(homoLys)-Gly-Asp-Cys-O-Pam Ⓡ
Cleavage of the peptide from the resin is achieved using HF/anisole (9:1 (v/v)) to form:

H                                                    H
Acetyl-Cys-Asn-(DiMeTzl)-(homoLys)-Gly-Asp-Cys-OH.

A cyclic structure is formed by formation of a disulfide bridge between the cysteine residues. The peptide is dissolved in 50-80% AcOH:H₂O at room temperature, and the solution stirred during rapid addition of a solution of iodine in AcOH to a final concentration of 2.25 mg/ml of iodine. After 1-2 hours reaction time, excess I₂ and AcOH are removed by rotary evaporation under vacuum and the aqueous solution containing the cyclized peptide is purified using preparative HPLC in 0.1% TFA H₂O-CH₃CN gradient at which stage the D- and L- diastereomers are separated by conventional means. The final TFA salt product is converted to HOAc salt by passing through an ion exchange column BioRad AG3-X4A (acetate cycle). The finished peptide is:

Acetyl-Cys-Asn-(DiMeTzl)-(homoLys)-Gly-Asp-Cys-OH.

As an alternative to formation of the disulfide by iodine oxidation, the free SH peptide is dissolved in 1-5% HOAc at a concentration of approximately 2 mg/ml and the solution is adjusted to approximately pH 7-8.5 with concentrated NH₄OH. Cyclization is accomplished under brisk stirring (preferably with a small piece of copper wire added to accelerate the reaction) during a period of 1-4 hours at 25°. The reaction mixture is then concentrated as before and product purified by preparative HPLC.

### Therapeutic Utility

Compounds of the invention may be administered to patients where prevention of thrombosis by inhibiting binding of fibrinogen to the platelet membrane glycoprotein complex IIb/IIIa receptor is desired. They are useful in surgery on peripheral arteries (arterial grafts, carotid endarterectomy) and in cardiovascular surgery where manipulation of arteries and organs, and/or the interaction of platelets with artificial surfaces, leads to platelet aggregation and consumption. The aggregated platelets may form thrombi and thromboemboli. Polypeptides of the invention may be administered to these surgical patients to prevent the formation of thrombi and thromboemboli.

Extracorporeal circulation is routinely used for cardiovascular surgery in order to oxygenate blood. Platelets adhere to surfaces of the extracorporeal circuit. Adhesion is dependent on the interaction between GPIIb/IIIa on the platelet membranes and fibrinogen adsorbed to the surface of the circuit. (Gluszko et al., Amer. J. Physiol., 1987, 252:H, pp 615-621). Platelets released from artificial surfaces show impaired hemostatic function. Polypeptides of the invention may be administered to prevent adhesion.

Other applications of these polypeptides include prevention of platelet thrombosis, thromboembolism and reocclusion during and after thrombolytic therapy and prevention of platelet thrombosis, thromboembolism and reocclusion after angioplasty of coronary and other arteries and after coronary artery bypass procedures. Polypeptides of the invention may also be used to prevent myocardial infarction.

These polypeptides may be administered by any convenient means which will result in its delivery into the blood stream in substantial amount including continuous intravenous or bolus injection or oral methods. Compositions of the invention include peptides of the invention and pharmacologically acceptable carriers, e.g. saline, at a pH level e.g. 7.4, suitable for achieving inhibition of platelet aggregation. They may be combined with thrombolytic agents such as plasminogen activators or streptokinase in order to inhibit platelet aggregation. They may also be combined with anticoagulants such as heparin, aspirin or warfarin. Intravenous administration is presently contemplated as the preferred administration route. They are soluble in water, and may therefore be effectively administered in solution.

In one exemplary application, a suitable amount of peptide is intravenously administered to a heart attack victim undergoing angioplasty. Administration occurs during or several minutes prior to angioplasty, and is in an amount sufficient to inhibit platelet aggregation, e.g. an amount which achieves a steady state plasma concentration of between about 0.05-30 »M per kilo, preferably between about 0.3-3 »m per kilo. When this amount is achieved, an infusion of between about 1-100 nM per kilo per min., preferably between about 10-30 nM per kilo per min. is maintained to inhibit platelet aggregation. Should the patient need to undergo bypass surgery, administration may be stopped immediately and will not cause complications during surgery that would be caused by other materials such as aspirin or monoclonal antibodies, the effects of which last hours after cessation of administration.

The present invention also includes a pharmaceutical composition comprising peptides of the present invention and tissue type plasminogen activator or streptokinase. The invention also includes a method for promoting thrombolysis and preventing reocclusion is a patient which comprises administering to the patient an effective amount of compositions of the invention.

The specific examples described above should not be interpreted as limiting the scope of the present invention.

## Claims (Claims for the following Contracting State(s): AT, BE, CH, DE, DK, FR, GB, IT, LI, LU, NL, SE)

1. A fibrinogen receptor antagonist compound which comprises the sequence
XX-Gly-Asp
wherein XX represents a synthetic alpha-amino acid containing a linear side chain defined as 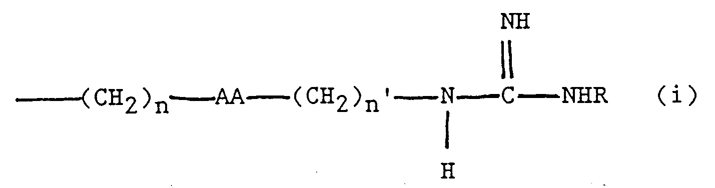 wherein n + n' is 3; AA is a single bond; and R is phenyl or benzyl;
or
―(CH₂)ₙ―AA―(CH₂)ₙ'―NHR (ii)
wherein:
n is 1,2,3 or 4;
n' is 2,3 or 4;
AA is an oxygen atom, a sulfur atom, or a single bond; and
R is H, C₁₋₆ alkyl, substituted or unsubstituted aryl, substituted or unsubstituted arylmethyl or substituted or unsubstituted cycloalkyl, provided that when AA is a single bond and R is H, then n+n' is not 4 or 3

2. A fibrinogen receptor antagonist of the formula: 
Z Z
XX-Gly-Asp
 wherein XX represents a synthetic alpha-amino acid containing a linear side chain
defined as 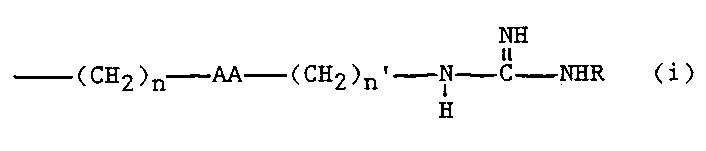 wherein n + n' is 3; AA is a single bond; and R is phenyl or benzyl;
or
―(CH₂)ₙ―AA―(CH₂)ₙ'―NHR (ii)
wherein:
n is 1,2,3 or 4;
n' is 2,3 or 4;
AA is an oxygen atom, a sulfur atom, or a single bond; and
R is H, C₁₋₆ alkyl, substituted or unsubstituted aryl, substituted or unsubstituted arylmethyl or substituted or unsubstituted cycloalkyl, provided that when AA is a single bond and R is H, then n+n' is not 4 or 3, and ZZ represents a sequence of 1, 2, 3 or 4 substituted or unsubstituted amino acids.

3. A compound of claim 2 wherein ZZ is 1, 2, 3 or 4 amino acids according to formulas I, II, III, IV or V: 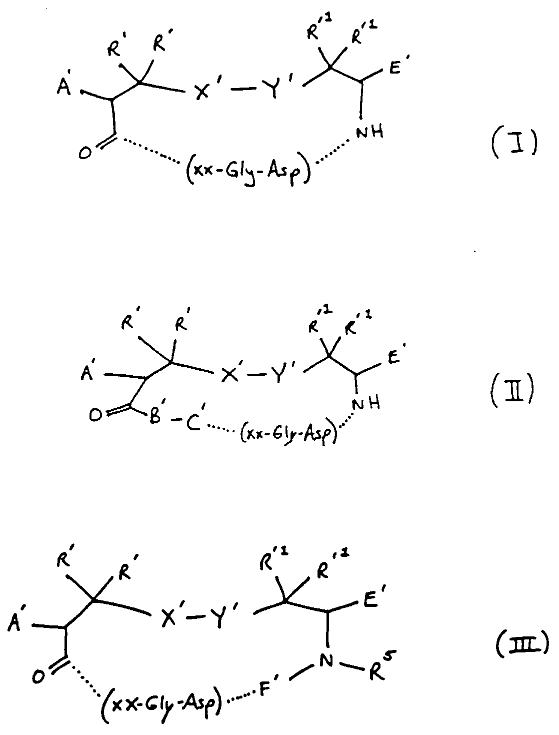 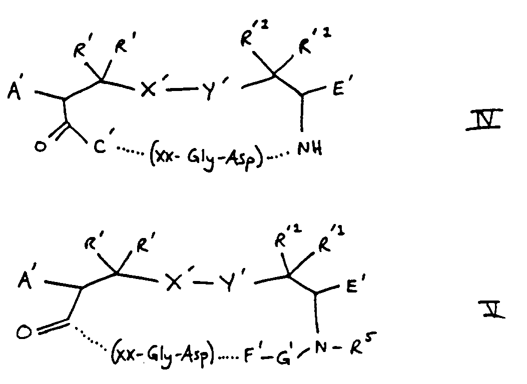 wherein
A' is H, acylamido, acylaminoacylamido, acylamino-N-methylaminoacyl-amido;
R' and R'¹ are independently H, methyl, ethyl or a lower alkyl group having 1 to 5 carbons;
X'-Y' is S-S, CH₂-S, S-CH₂, CH₂CH₂, CH₂,CH₂CH₂CH₂, CH₂-S-S, CH₂-S-S-CH₂, S-S-CH₂; and
E' is H, COOH, CONH₂, CONHR², CONR³R⁴, CH₂OH,CO₂R²,CH₃ wherein R² is an alkyl group having 1 to 4 carbon atoms, R³R⁴ is an alkyl group having 1 to 4 carbon atoms or NR³R⁴ is a secondary amino acid,
or 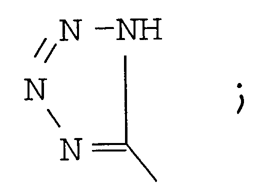 B' is a D- or L- α-amino acid;
C' is a D- or L- secondary α-amino acid or a D- or L-primary amino acid;
F' is an L- α-mino acid;
G' is a D- or L- α-amino acid, secondary cyclic amino acid, or N-methyl amino acid; and
R5 is H or methyl.

4. A compound of claim 2 which is

Ac-Cys-Asn-(DiMeTzl)-(homoLys)-Gly-Asp-Cys-OH

5. A compound of Claim 2 which is
c(Aha-(homoLys)-Gly-Asp-Trp-Pro)

6. A composition for inhibiting fibrinogen-dependent platelet aggregation in a mammal comprising a compound of claim 1 and a pharmaceutically acceptable carrier.

7. The use of a compound of Claim 1 for the preparation of a medicament suitable for inhibiting fibrinogen binding to mammalian platelets.

8. A composition for inhibiting fibrinogen-dependent platelet aggregation in a mammal comprising a compound of claim 2 and a pharmaceutically acceptable carrier.

9. The use of a compound of Claim 2 for the preparation of a medicament suitable for inhibiting fibrinogen binding to mammalian platelets.

## Claims (Claims for the following Contracting State(s): ES, GR)

1. A process for preparing a fibrinogen receptor antagonist of the formula
XX-Gly-Asp
wherein XX represents a synthetic alpha-amino acid containing a linear side chain defined as 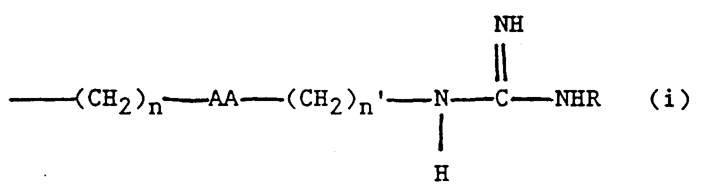 wherein n + n' is 3; AA is a single bond; and R is phenyl or benzyl;
or
―(CH₂)ₙ―AA―(CH₂)ₙ'―NHR (ii)
wherein:
n is 1,2,3 or 4;
n' is 2,3 or 4;
AA is an oxygen atom, a sulfur atom, or a single bond; and
R is H, C₁₋₆ alkyl, substituted or unsubstituted aryl, substituted or unsubstituted arylmethyl or substituted or unsubstituted cycloalkyl, provided that when AA is a single bond and R is H, then n+n' is not 4 or 3, which comprises:
i) binding an amino group attached to the alpha carbon of a first amino acid to a carboxyl group attached to the alpha carbon of a second amino acid in the presence of a condensing agent, such that a peptide bond forms and the second amino acid is termed the terminal amino acid and all reactive groups of the amino acids not participating directly in peptide bond formation are bound to a protecting group or resin;
ii) releasing the protecting group to the amino group attached to the alpha carbon of the terminal amino acid;
iii) binding the amino group of the terminal amino acid released by step ii) to a carboxyl group attached to the alpha carbon of an amino acid in the presence of a condensing agent, such that a peptide bond forms and the amino acid added is termed the terminal amino acid and all reactive groups of the amino acids not participating directly in peptide bond formation are bound to a protecting group or resin; and
iv) repeating steps ii) and iii) until a peptide chain of the desired length is achieved.

2. A process as claimed in claim 1 for preparing a fibrinogen receptor antagonist of the formula: 
Z Z
XX-Gly-Asp
 wherein XX represents a synthetic alpha-amino acid containing a linear side chain
defined as 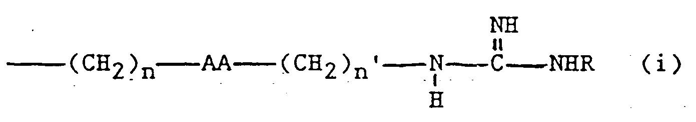 wherein n + n' is 3; AA is a single bond; and R is phenyl or benzyl;
or
―(CH₂)ₙ―AA―(CH₂)ₙ'―NHR (ii)
wherein:
n is 1,2,3 or 4;
n' is 2,3 or 4;
AA is an oxygen atom, a sulfur atom, or a single bond; and
R is H, C₁₋₆ alkyl, substituted or unsubstituted aryl, substituted or unsubstituted arylmethyl or substituted or unsubstituted cycloalkyl, provided that when AA is a single bond and R is H, then n+n' is not 4 or 3,
and ZZ represents a sequence of 1, 2, 3 or 4 substituted or unsubstituted amino acids.

3. A process according to claim 2 for preparing a compound wherein
ZZ is 1, 2, 3 or 4 amino acids according to formulas I, II, III, IV or V: 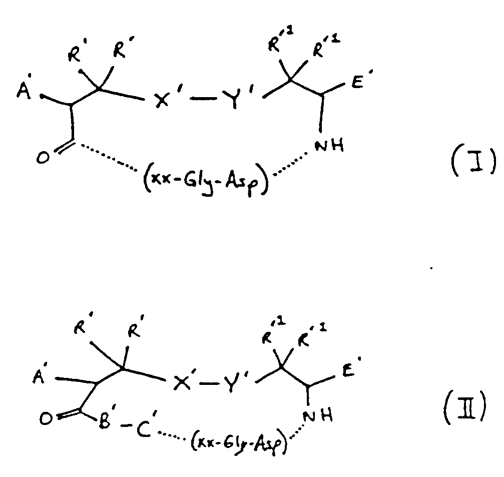 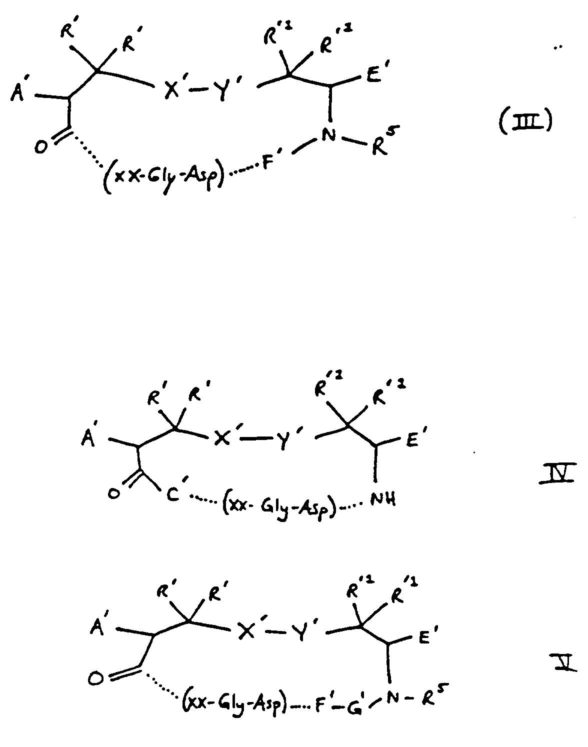 wherein
A' is H, acylamido, acylaminoacylamido, acylamino-N-methylaminoacyl-amido;
R¹ and R'¹ are independently H, methyl, ethyl or a lower alkyl group having 1 to 5 carbons;
X'-Y' is S-S, CH₂-S, S-CH₂, CH₂CH₂, CH₂,CH₂CH₂CH₂, CH₂-S-S, CH₂-S-S-CH₂, S-S-CH₂; and
E' is H, COOH, CONH₂, CONHR², CONR³R⁴, CH₂OH,CO₂R²,CH₃ wherein R² is an alkyl group having 1 to 4 carbon atoms, R³R⁴ is an alkyl group having 1 to 4 carbon atoms or NR³R⁴ is a secondary amino acid,
or 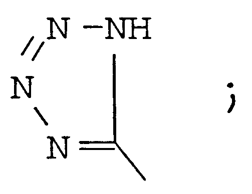 B' is a D- or L- α-amino acid;
C' is a D- or L- secondary α-amino acid or a D- or L-primary amino acid;
F' is an L- α-mino acid;
G' is a D- or L- α-amino acid, secondary cyclic amino acid, or N-methyl amino acid; and
R5 is H or methyl.

4. A process according to claim 2 for preparing a compound which is

Ac-Cys-Asn-(DiMeTzl)-(homoLys)-Gly-Asp-Cys-OH

5. A process according to claim 2 for preparing a compound which is
c(Aha-(homoLys)-Gly-Asp-Trp-Pro)

## Patentansprüche (Patentansprüche für folgende(n) Vertragsstaat(en): AT, BE, CH, DE, DK, FR, GB, IT, LI, LU, NL, SE)

1. Fibrinogenrezeptor-Antagonistenverbindung, welche die Sequenz
XX-Gly-Asp
umfaßt, worin XX eine synthetische α-Aminosäure darstellt, die eine lineare Seitenkette enthält, die definiert ist als 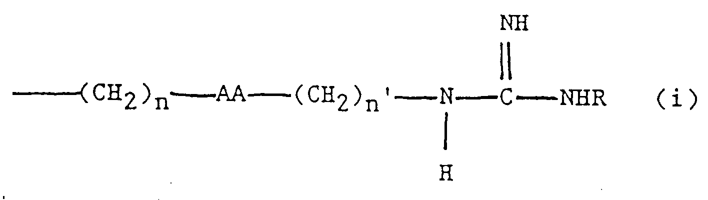 worin n + n' 3 ist, AA eine Einfachbindung darstellt und R für Phenyl oder Benzyl steht,
oder als
―(CH₂)ₙ―AA―(CH₂)ₙ'―NHR (ii)
worin:
n für 1, 2, 3 oder 4 steht,
n' für 2, 3 oder 4 steht,
AA ein Sauerstoffatom, ein Schwefelatom oder eine Einfachbindung darstellt und
R für H, C₁₋₆-Alkyl, substituiertes oder unsubstituiertes Aryl, substituiertes oder unsubstituiertes Arylmethyl oder substituiertes oder unsubstituiertes Cycloalkyl steht, mit der Maßgabe, dar, wenn AA eine Einfachbindung ist und R für H steht, n + n' dann nicht 4 oder 3 ist.

2. Fibrinogenrezeptor-Antagonist der Formel: 
Z Z
XX-Gly-Asp
 worin XX eine synthetische α-Aminosäure darstellt, die eine lineare Seitenkette enthält, die definiert ist als 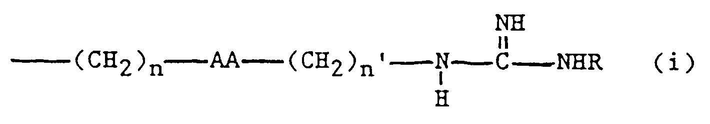 worin n + n' 3 ist, AA für eine Einfachbindung steht und R für Phenyl oder Benzyl steht,
oder als
―(CH₂)ₙ―AA―(CH₂)ₙ―NHR (ii)
worin:
n 1, 2, 3 oder 4 bedeutet,
n' für 2, 3 oder 4 steht,
AA ein Sauerstoffatom, ein Schwefelatom oder eine Einfachbindung darstellt und
R für H, C₁₋₆-Alkyl, substituiertes oder unsubstituiertes Aryl, substituiertes oder unsubstituiertes Arylmethyl oder substituiertes oder unsubstituiertes Cycloalkyl steht, mit der Maßgabe, daß, wenn AA eine Einfachbindung ist und R für H steht, n + n' dann nicht 4 oder 3 ist, und
ZZ für eine Sequenz von 1, 2, 3 oder 4 substituierten oder unsubstituierten Aminosäuren steht.

3. Verbindung nach Anspruch 2, worin ZZ für 1, 2, 3 oder 4 Aminosäuren gemäß den Formeln I, II, III, IV oder V steht: 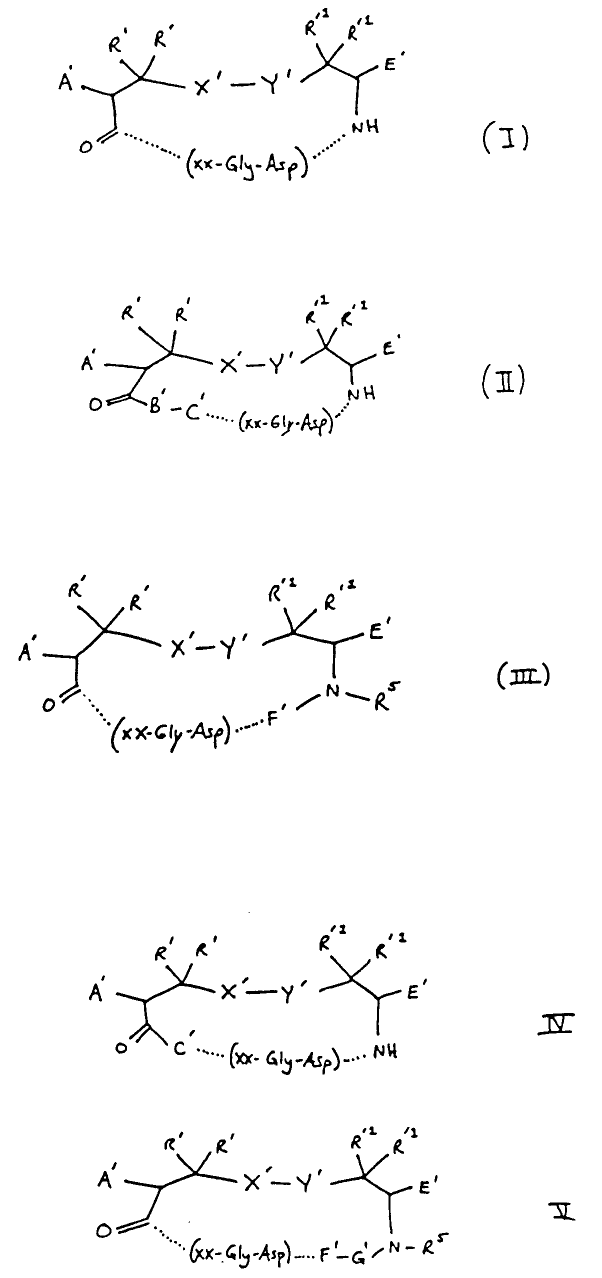 worin
A' für H, Acylamido, Acylaminoacylamido, Acylamino-N-methylaminoacylamido steht,
R' und R'¹ unabhängig für H, Methyl, Ethyl oder eine Niedrigalkylgruppe mit 1 bis 5 Kohlenstoffatomen stehen,
X'-Y' für S-S, CH₂-S, S-CH₂, CH₂CH₂, CH₂, CH₂CH₂CH₂, CH₂-S-S, CH₂-S-S-CH₂, S-S-CH₂ steht und
E' für H, COOH, CONH₂, CONHR², CONR³R⁴, CH₂OH, CO₂R², CH₃ steht, worin R² für eine Alkylgruppe mit 1 bis 4 Kohlenstoffatomen steht, R³R⁴ eine Alkylgruppe mit 1 bis 4 Kohlenstoffatomen darstellt oder NR³R⁴ eine sekundäre Aminosäure oder 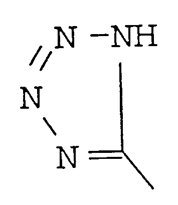 darstellt,
B' für eine D- oder L-α-Aminosäure steht,
C' für eine sekundäre D- oder L-α-Aminosäure oder eine primäre D- oder L-Aminosäure steht,
F' für eine L-α-Aminosäure steht,
G' für eine D- oder L-α-Aminosäure, eine sekundäre cyclische Aminosäure oder eine N-Methylaminosäure steht und
R⁵ H oder Methyl bedeutet.

4. Verbindung nach Anspruch 2, die Ac-Cys-Asn-(DiMeTzl)-(homoLys)-Gly-Asp-Cys-OH ist.

5. Verbindung nach Anspruch 2, die
c(Aha-(homoLys)-Gly-Asp-Trp-Pro)
ist.

6. Präparat zur Hemmung der Fibrinogen-abhängigen Thrombozytenaggregation bei einem Säuger, umfassend eine Verbindung nach Anspruch 1 und einen pharmazeutisch verträglichen Trägerstoff.

7. Verwendung einer Verbindung nach Anspruch 1 zur Herstellung eines Medikaments, das zur Hemmung des Bindens von Fibrinogen an Säuger-Thrombozyten geeignet ist.

8. Präparat zur Hemmung der Fibrinogen-abhängigen Thrombozytenaggregation bei einem Säuger, umfassend eine Verbindung nach Anspruch 2 und einen pharmazeutisch verträglichen Trägerstoff.

9. Verwendung einer Verbindung nach Anspruch 2 zur Herstellung eines Medikaments, das zur Hemmung des Bindens von Fibrinogen an Säuger-Thrombozyten geeignet ist.

## Patentansprüche (Patentansprüche für folgende(n) Vertragsstaat(en): ES, GR)

1. Verfahren zur Herstellung eines Fibrinogenrezeptor-Antagonisten der Formel
XX-Gly-Asp
worin XX eine synthetische α-Aminosäure darstellt, die eine lineare Seitenkette enthält, die definiert ist als 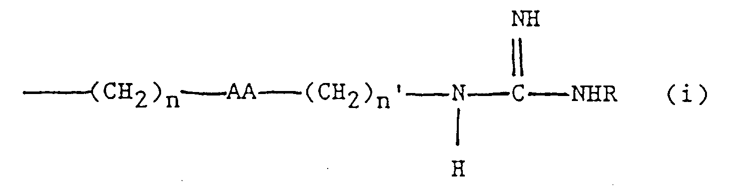 worin n + n' 3 ist, AA eine Einfachbindung darstellt und R für Phenyl oder Benzyl steht,
oder als
―(CH₂)ₙ―AA―(CH₂)ₙ'―NHR (ii)
worin:
n für 1, 2, 3 oder 4 steht,
n' für 2, 3 oder 4 steht,
AA ein Sauerstoffatom, ein Schwefelatom oder eine Einfachbindung darstellt und
R für H, C₁₋₆-Alkyl, substituiertes oder unsubstituiertes Aryl, substituiertes oder unsubstituiertes Arylmethyl oder substituiertes oder unsubstituiertes Cycloalkyl steht, mit der Maßgabe, daß, wenn AA eine Einfachbindung ist und R für H steht, n + n' dann nicht 4 oder 3 ist, welches umfaßt:
i) Binden einer an den α-Kohlenstoff einer ersten Aminosäure angeknüpften Aminogruppe an eine an den α-Kohlenstoff einer zweiten Aminosäure angeknüpften Carboxygruppe in Gegenwart eines Kondensationsmittels, so daß sich eine Peptidbindung bildet und die zweite Aminosäure als terminale Aminosäure bezeichnet wird und alle reaktiven Gruppen der Aminosäuren, die nicht direkt an der Bildung der Peptidbindung teilhaben an eine Schutzgruppe oder ein Harz gebunden sind,
(ii) Freisetzen der Schutzgruppe an der an den α-Kohlenstoff der teminalen Aminosäure angeknüpften Aminogruppe,
(iii) Binden der in Stufe (ii) freigesetzten Aminogruppe der terminalen Aminosäure an eine an den α-Kohlenstoff einer Aminosäure angeknüpften Carboxygruppe in Gegenwart eines Kondensationsmittels, so daß sich eine Peptidbindung bildet und die zweite Aminosäure als terminale Aminosäure bezeichnet wird und alle reaktiven Gruppen der Aminosäuren, die nicht direkt an der Bildung der Peptidbindung teilhaben an eine Schutzgruppe oder ein Harz gebunden sind, und
(iv) Wiederholen der Stufen ii) und iii), bis eine Peptidkette der gewünschten Länge erreicht ist.

2. Verfahren nach Anspruch 1 zur Herstellung eines Fibrinogenrezeptor-Antagonisten der Formel: 
Z Z
XX-Gly-Asp
 worin XX eine synthetische α-Aminosäure darstellt, die eine lineare Seitenkette enthält, die definiert ist als 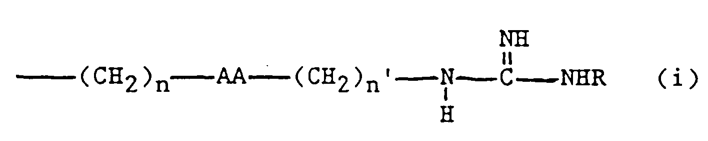 worin n + n' 3 ist, AA für eine Einfachbindung steht und R für Phenyl oder Benzyl steht,
oder als
―(CH₂)ₙ―AA―(CH₂)ₙ'―NHR (ii)
worin:
n 1, 2, 3 oder 4 bedeutet,
n' für 2, 3 oder 4 steht,
AA ein Sauerstoffatom, ein Schwefelatom oder eine Einfachbindung darstellt und
R für H, C₁₋₆-Alkyl, substituiertes oder unsubstituiertes Aryl, substituiertes oder unsubstituiertes Arylmethyl oder substituiertes oder unsubstituiertes Cycloalkyl steht, mit der Maßgabe, daß, wenn AA eine Einfachbindung ist und R für H steht, n + n' dann nicht 4 oder 3 ist, und
ZZ für eine Sequenz von 1, 2, 3 oder 4 substituierten oder unsubstituierten Aminosäuren steht.

3. Verfahren nach Anspruch 2 zur Herstellung einer Verbindung, worin ZZ für 1, 2, 3 oder 4 Aminosäuren gemäß den Formeln I, II, III, IV oder V steht: 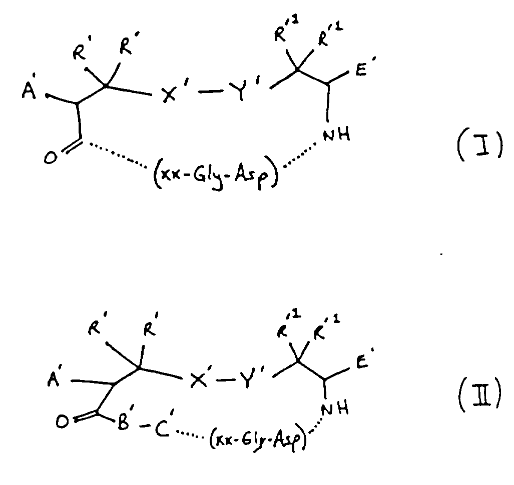 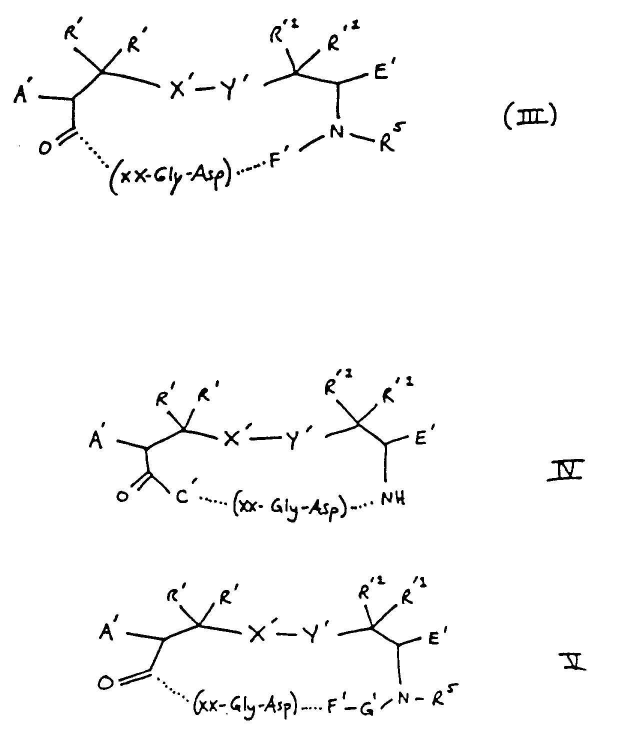 worin
A' für H, Acylamido, Acylaminoacylamido, Acylamino-N-methylaminoacylamido steht,
R' und R'¹ unabhängig für H, Methyl, Ethyl oder eine Niedrigalkylgruppe mit 1 bis 5 Kohlenstoffatomen stehen,
X'-Y' für S-S, CH₂-S, S-CH₂, CH₂CH₂, CH₂, CH₂CH₂CH₂, CH₂-S-S, CH₂-S-S-CH₂, S-S-CH₂ steht und
E' für H, COOH, CONH₂, CONHR², CONR³R⁴, CH₂OH, CO₂R², CH₃ steht, worin R² für eine Alkylgruppe mit 1 bis 4 Kohlenstoffatomen steht, R³R⁴ eine Alkylgruppe mit 1 bis 4 Kohlenstoffatomen darstellt oder NR³R⁴ eine sekundäre Aminosäure oder 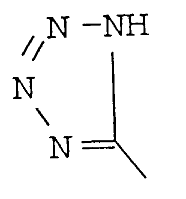 darstellt,
B' für eine D- oder L-α-Aminosäure steht,
C' für eine sekundäre D- oder L-α-Aminosäure oder eine primäre D- oder L-Aminosäure steht,
F' für eine L-α-Aminosäure steht,
G' für eine D- oder L-α-Aminosäure, eine sekundäre cyclische Aminosäure oder eine N-Methylaminosäure steht und
R⁵ H oder Methyl bedeutet.

4. Verfahren nach Anspruch 2 zur Herstellung einer Verbindung, die Ac-Cys-Asn-(DiMeTzl)-(homoLys)-Gly-Asp-Cys-OH ist.

5. Verfahren nach Anspruch 2 zur Herstellung einer Verbindung, die
c(Aha-(homoLys)-Gly-Asp-Trp-Pro)
ist.

## Revendications (Revendications pour l'(les) Etat(s) contractant(s) suivant(s): AT, BE, CH, DE, DK, FR, GB, IT, LI, LU, NL, SE)

1. Composé antagoniste du récepteur de fibrinogène qui comprend la séquence
XX-Gly-Asp
dans laquelle XX représente un acide alpha-aminé synthétique contenant une chaîne latérale linéaire définie par : 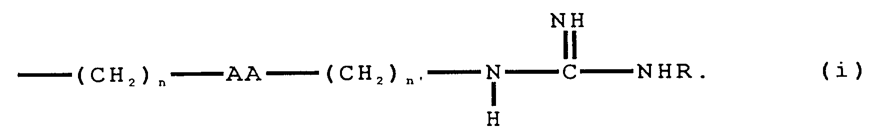 dans laquelle n + n' est 3; AA est un pont unique; et R est un phényle ou un benzyle;
ou
―(CH₂)ₙ―AA―(CH₂)ₙ'―NHR (ii)
dans laquelle :
n est 1, 2, 3 ou 4;
n' est 2, 3 ou 4;
AA est un atome d'oxygène, un atome de soufre, ou un pont unique; et
R est H, C₁₋₆ alkyle, un aryle substitué ou non-substitué, un arylméthyle substitué ou non-substitué ou un cycloalkyle substitué ou non-substitué, à condition que lorsque AA est un pont unique et R est H, alors n + n' n'est ni 4 ni 3.

2. Antagoniste du récepteur de fibrinogène de formule : 
Z Z
XX-Gly-Asp
 dans laquelle XX représente un acide α-aminé synthétique contenant une chaîne latérale linéaire définie par : 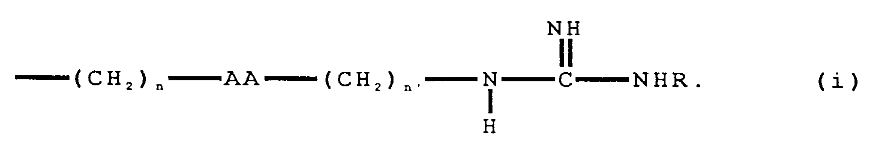 dans laquelle n + n' est 3; AA est un pont unique; et R est un phényle ou un benzyle;
ou
―(CH₂)ₙ―AA―(CH₂)ₙ'―NHR (ii)
dans laquelle :
n est 1, 2, 3 ou 4;
n' est 2, 3 ou 4;
AA est un atome d'oxygène, un atome de soufre, ou un pont unique; et
R est H, C₁₋₆ alkyle, un aryle substitué ou non-substitué, un arylméthyle substitué ou non-substitué ou un cycloalkyle substitué ou non-substitué, à condition que lorsque AA est un pont unique et R est H, alors n + n' n'est ni 4 ni 3, et ZZ représente une séquence de 1, 2, 3 ou 4 acides aminés substitués ou non-substitués.

3. Composé selon la revendication 2 dans lequel ZZ est 1, 2, 3 ou 4 acides aminés selon les formules I, II, III, IV ou V : 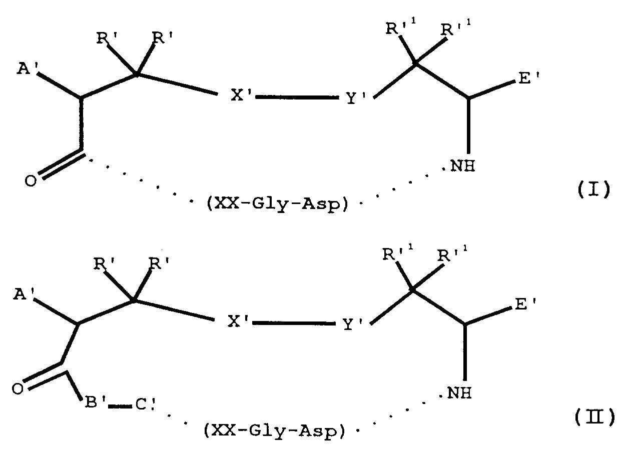 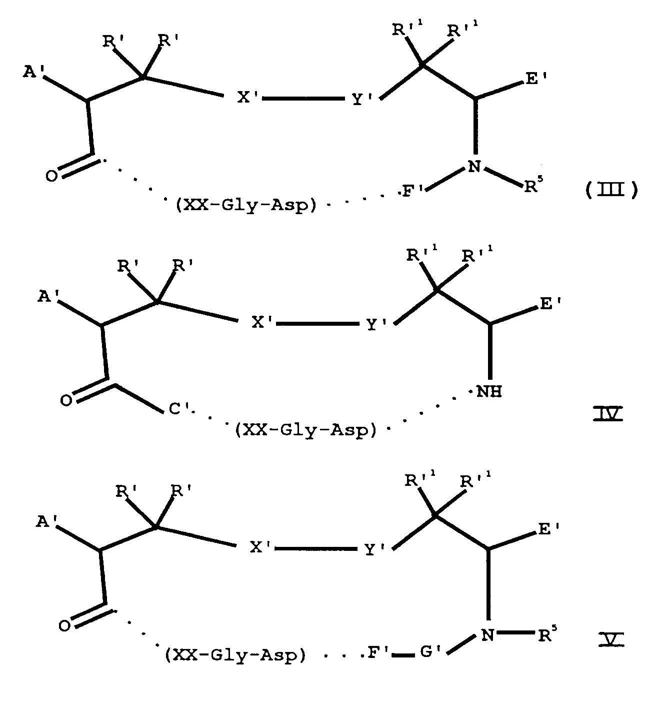 où
A' est H, acylamide, acylaminoacylamide, acylamino-N-méthylamino-acylamide;
R' et R'¹ sont indépendamment H, un méthyle, un éthyle ou un groupe alkyle inférieur ayant 1 à 5 carbones;
X'-Y' est S-S, CH₂-S, S-CH₂, CH₂CH₂, CH₂, CH₂CH₂CH₂, CH₂-S-S, CH₂-S-S-CH₂, S-S-CH₂; et
E' est H, COOH, CONH₂, CONHR², CONR³R⁴, CH₂OH, CO₂R², CH₃ où R² est un groupe alkyle ayant 1 à 4 atomes de carbone, R³R⁴ est un groupe alkyle ayant 1 à 4 atomes de carbone ou NR³R⁴ est un acide aminé secondaire, ou 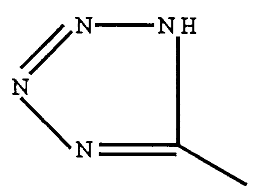 B' est un acide α-aminé D ou L;
C' est un acide α-aminé secondaire D ou L ou un acide aminé primaire D ou L;
F' est un acide α-aminé L
G' est un acide α-aminé L ou D; un acide aminé cyclique secondaire, ou un acide aminé N-méthyle; et
R⁵ est H ou un méthyle.

4. Composé selon la revendication 2 qui est

Ac-Cys-Asn-(DiMeTzl)-(homoLys)-Gly-Asp-Cys-OH.

5. Composé selon la revendication 2 qui est c(Aha-(homoLys)-Gly-Asp-Trp-Pro).

6. Composition pour inhiber l'agrégation des plaquettes dépendante du fibrinogène dans un mammifère comprenant un composé selon la revendication 1, et un support acceptable de manière pharmaceutique.

7. Utilisation d'un composé selon la revendication 1, pour la préparation d'un médicament approprié à l'inhibition de la liaison du fibrinogène aux plaquettes de mammifères.

8. Composition pour inhiber l'agrégation des plaquettes dépendante du fibrinogène dans un mammifère comprenant un composé selon la revendication 2, et un support acceptable de manière pharmaceutique.

9. Utilisation d'un composé selon la revendication 2, pour la préparation d'un médicament approprié à l'inhibition de la liaison du fibrinogène aux plaquettes de mammifères.

## Revendications (Revendications pour l'(les) Etat(s) contractant(s) suivant(s): ES, GR)

1. Procédé pour la préparation d'un antagoniste du récepteur de fibrinogène de formule
XX-Gly-Asp
dans laquelle XX représente un acide alpha-aminé synthétique contenant une chaîne latérale linéaire définie par : 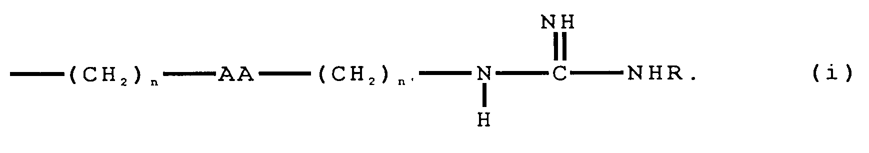 dans laquelle n + n' est 3; AA est un pont unique; et R est un phényle ou un benzyle;
ou
―(CH₂)ₙ―AA―(CH₂)ₙ'―NHR (ii)
dans laquelle :
n est 1, 2, 3 ou 4;
n' est 2, 3 ou 4;
AA est un atome d'oxygène, un atome de soufre, ou un pont unique; et
R est H, C₁₋₆ alkyle, un aryle substitué ou non-substitué, un arylméthyle substitué ou non-substitué ou un cycloalkyle substitué ou non-substitué, à condition que lorsque AA est un pont unique et R est H, alors n + n' n'est ni 4 ni 3 qui consiste à :
i) lier un groupe amine attaché au carbone alpha d'un premier acide aminé à un groupe carboxyle attaché au carbone alpha d'un second acide aminé en présence d'un agent condensant, de telle sorte qu'un pont peptidique se forme et que le second acide aminé devienne l'acide aminé terminal et que tous les groupes réactifs des acides aminés ne participant pas directement à la formation du pont peptidique sont liés à un groupe protecteur ou à une résine;
ii) libérer le groupe protégeant le groupe amine attaché au carbone alpha de l'acide aminé terminal;
iii) lier le groupe amine de l'acide aminé terminal libéré par l'étape ii) à un groupe carboxyle attaché au carbone alpha d'un acide aminé en présence d'un agent condensant, de telle sorte qu'un pont peptidique se forme et que l'acide aminé ajouté devienne l'acide aminé terminal et que tous les groupes réactifs des acides aminés ne participant pas directement à la formation du pont peptidique sont liés à un groupe protecteur ou à une résine; et
iv) répéter les étapes ii) et iii) jusqu'à ce qu'une chaîne peptidique de la longueur désirée soit terminée.

2. Procédé comme revendiqué selon la revendication 1 pour la préparation d'un antagoniste du récepteur de fibrinogène de formule : 
Z Z
XX-Gly-Asp
 dans laquelle XX représente un acide α-aminé synthétique contenant une chaîne latérale définie : 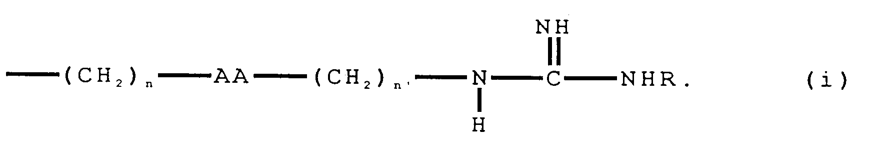 où n + n' est 3; AA est un pont unique; et R est un phényle ou un benzyle;
ou
―(CH₂)ₙ―AA―(CH₂)_{n'}―NHR (ii)
où :
n est 1, 2, 3 ou 4;
n' est 2, 3 ou 4;
AA est un atome d'oxygène, un atome de soufre, ou un pont unique; et
R est H, C₁₋₆ alkyle, un aryle substitué ou non-substitué, un arylméthyle substitué ou non-substitué ou un cycloalkyle substitué ou non-substitué, à condition que lorsque AA est un pont unique et R est H, alors n + n' n'est ni 4 ni 3, et ZZ représente une séquence de 1, 2, 3 ou 4 acides aminés substitués ou non-substitués.

3. Procédé selon la revendication 2 pour la préparation d'un composé où ZZ est 1, 2, 3 ou 4 acides aminés selon les formules I, II, III, IV ou V : 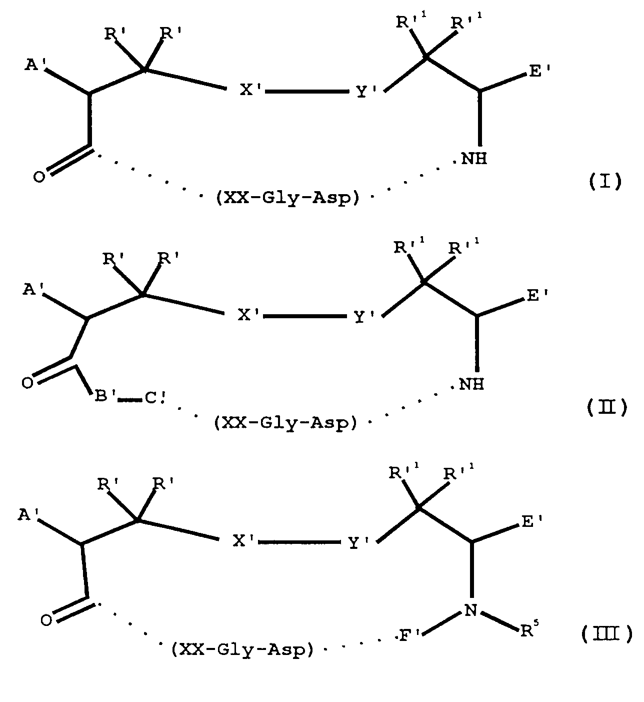 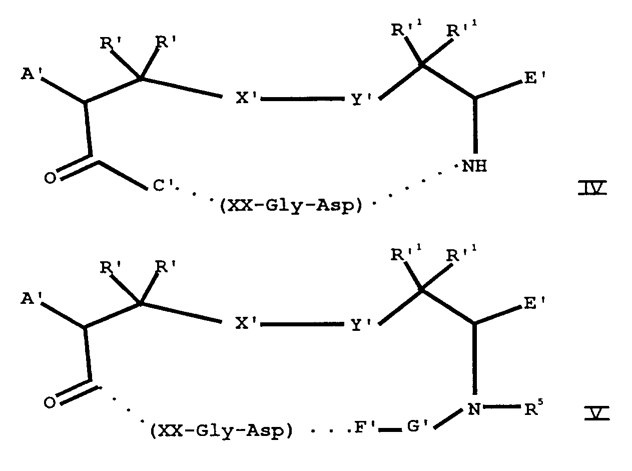 où
A' est H, acylamide, acylaminoacylamide, acylamino-N-méthylamino-acylamide;
R' et R'¹ sont indépendamment H, un méthyle, un éthyle ou un groupe alkyle inférieur ayant 1 à 5 carbones;
X'-Y' est S-S, CH₂-S, S-CH₂, CH₂CH₂, CH₂, CH₂CH₂CH₂, CH₂-S-S, CH₂-S-S-CH₂, S-S-CH₂; et
E' est H, COOH, CONH₂, CONHR², CONR³R⁴, CH₂OH, CO₂R², CH₃ où R² est un groupe alkyle ayant 1 à 4 atomes de carbone, R³R⁴ est un groupe alkyle ayant 1 à 4 atomes de carbone ou NR³R⁴ est un acide aminé secondaire, ou 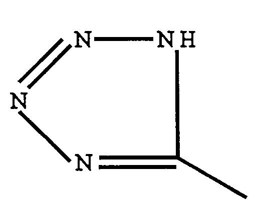 B' est un acide α-aminé D ou L;
C' est un acide α-aminé secondaire D ou L ou un acide aminé primaire D ou L;
F' est un acide α-aminé L
G' est un acide α-aminé L ou D; un acide aminé cyclique secondaire, ou un acide aminé N-méthyle; et
R⁵ est H ou méthyle.

4. Procédé selon la revendication 2 pour la préparation d'un composé qui est

Ac-Cys-Asn-(DiMeTzl)-(homoLys)-Gly-Asp-Cys-OH.

5. Procédé selon la revendication 2 pour la préparation d'un composé qui est c(Aha-(homoLys)-Gly-Asp-Trp-Pro).
